(19) **Europäisches Patentamt**
**European Patent Office**
**Office européen des brevets**

(11) **EP 3 555 258 B1**

(12) **EUROPEAN PATENT SPECIFICATION**

(45) Date of publication and mention
of the grant of the patent:
**01.04.2020  Bulletin 2020/14**

(51) Int Cl.:
*C12M 1/107* *(2006.01)*    *C12M 1/00* *(2006.01)*
*C12M 1/12* *(2006.01)*

(21) Application number: **17816689.8**

(86) International application number:
**PCT/EP2017/082215**

(22) Date of filing: **11.12.2017**

(87) International publication number:
**WO 2018/108810 (21.06.2018 Gazette 2018/25)**

(54) **MEMBRANE BIOREACTOR FOR BIOLOGICAL UPGRADING OF BIOGAS AND CONVERSION OF CO2 TO METHANE**

MEMBRAN-BIOREAKTOR ZUR BIOLOGISCHEN AUFBEREITUNG VON BIOGAS UND ZUR UMWANDLUNG VON CO2 ZU METHAN

BIORÉACTEUR À MEMBRANE POUR VALORISATION BIOLOGIQUE DE BIOGAZ ET DE CONVERSION DE CO2 EN MÉTHANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2016  EP 16203980**

(43) Date of publication of application:
**23.10.2019  Bulletin 2019/43**

(73) Proprietor: **Syddansk Universitet**
**5230 Odense M (DK)**

(72) Inventors:
• **NORDDAHL, Birgir**
**5750 Ringe (DK)**
• **TIRUNEHE, Gossaye, Weldegiorgis**
**5240 Odense NØ (DK)**

(74) Representative: **Plougmann Vingtoft a/s**
**Strandvejen 70**
**2900 Hellerup (DK)**

(56) References cited:
**US-A- 5 779 897        US-A1- 2009 286 296**
**US-A1- 2014 342 426**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field of the invention

The present invention relates to an improved biogas production facility. In particular the present invention relates to an improved biogas upgrading production facility, which produces a biogas with a high content of methane.

### Background of the invention

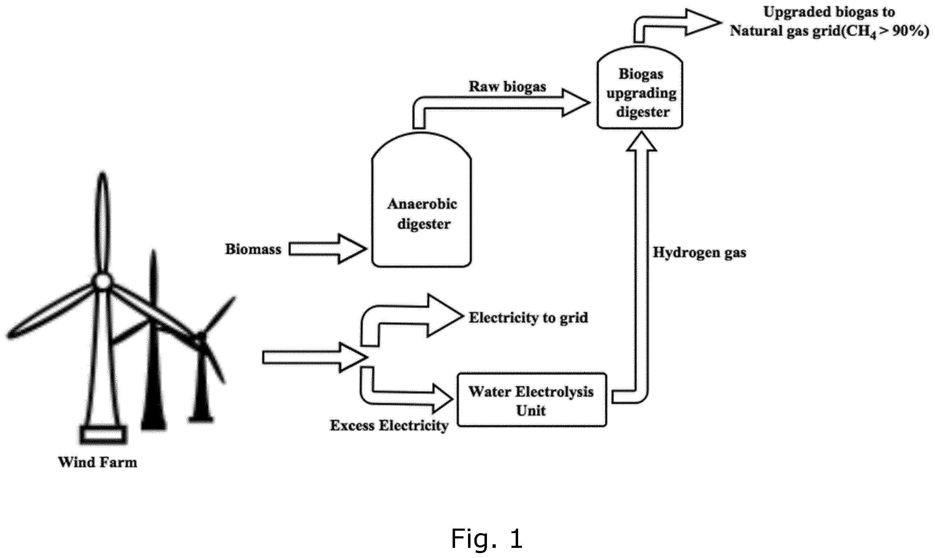

In several countries (including Denmark) renewable energy technology development, particularly wind power, solar power and biomass derived biogas production, is increasing.
As the contribution of renewable energy sources to the grid electricity pool increases, smooth balancing of this inherently intermittent supply with demand becomes a challenge for grid operators. In fact, as the proportion increases, grid balancing issues become serious resulting in the ultimate curbing of wind power. An innovative approach of integrating power to a gas process with anaerobic biotechnology offers the flexibility needed in energy systems to ensure long term, large scale storage of excess wind power in the form of chemical energy (natural gas quality biogas) using the existing natural gas pipe network (Figure 1).
Power-to-Gas (P2G) is a technological solution of converting excess wind energy to hydrogen gas using rapid response water electrolyzers. Some grid operators allow controlled injection of produced hydrogen into the existing natural gas transmission and distribution network. However, there are serious concerns over the safety risk of hydrogen mix and its potential impact on durability and integrity of the pipe materials.
The other attractive and novel approach is to direct the hydrogen to anaerobic digesters where it would be biochemically reacted with carbon dioxide present in biogas to produce natural gas quality upgraded biogas that can directly be injected to the gas network. The advanced natural gas network in e.g. Denmark offers vast capacity to store excess wind power as methane. There are different biogas upgrading technologies available, e.g. achieve the upgrading by removing carbon dioxide from biogas gas through various physicochemical processes.
While removing $CO_2$, some methane gas will also be removed that ultimately contributes to greenhouse gas emission. However, the new biological biogas upgrading process deploy the hydrogen supplied from P2G plant and microbiologically react it with carbon dioxide in anaerobic reactor resulting in simultaneous biogas enhancement and upgrading to natural gas quality.
US 2014/342426 relates to an anaerobic process for biogas upgrading and hydrogen utilization comprising the use of acidic waste as co-substrate. In this process, $H_2$ and $CO_2$ will be converted to $CH_4$, which will result in lower $CO_2$ content in the biogas.
US 2009/286296 describes a submerged membrane supported bioreactor for anaerobic conversion of gas into liquid products.
US 5 779 897 relates to separator module containing a bundle of hollow fiber membranes.
Hence, an improved biogas upgrading system would be advantageous, and in particular a more efficient and/or reliable biogas upgrading system would be advantageous.

### Summary of the invention

Introducing gaseous substrates hydrogen and carbon dioxide as electron donor and carbon source, poses its own technical challenge. The gaseous components $H_2$ and $CO_2$ needs to be efficiently and economically dissolved in the liquid medium and transferred to anaerobic microbial communities that convert them to methane. The stoichiometric equation of the biochemical reaction is:

$$4H_2 + CO_2 \rightarrow CH4 + 2H_2O \quad \Delta G^O = -130.7 \text{ kJ/mol}$$

Thus, 4 moles of sparsely soluble $H_2$ gas has to dissolve for each mole of methane gas produced. Furthermore, the hydrogenotrophic archaea microbial groups responsible for these bio-reactions, generate very little metabolic energy from the bioconversions. Consequently, they grow very slowly and often continue the conversions during the non-growth phase of their life cycle to gain metabolic energy for their maintenance. Therefore, the bioreactor to be employed for this process should be capable of achieving higher rates of gas liquid mass transfer while retaining sufficient active microbial density within the reactor.
Cell retention by formation of biofilms is a very good and often inexpensive way to increase cell density in the bioreactors. This requires a solid matrix with large surface area for the cells to colonize and form a biofilm that contains the metabolizing cells in a matrix of extracellular polymeric substance that the cells secret. Tubular microporous polymeric membranes stand out as ideal materials for meeting the requirements of high gas liquid mass transfer rate and retention of sufficient cell density. Moreover, the possibility of arranging tubular membranes in modular units having larger surface area for microbial biofilm attachment within a compact volume makes these bioreactors suitable for the process.

Thus, an object of the present invention relates to the provision of improved biogas upgrading systems.

Thus, one aspect of the invention relates to a system (100) for increasing the methane content of biogas, the system comprising

- a biogas reactor (1) capable of generating biogas; and

- a membrane biofilm reactor (9) arranged for converting carbon dioxide ($CO_2$) in the biogas and supplied hydrogen ($H_2$) into methane ($CH_4$), the membrane biofilm reactor being arranged for comprising a biofilm of methanogens (21) on or in, the membrane (20), said membrane comprising a first side and a second side;

wherein the membrane biofilm reactor (9) is positioned separately and outside of the biogas reactor (1), the biogas reactor being in fluid connection with the membrane biofilm reactor (9) by:

  ▪ a fluid intake line (6) for circulating fluid from the biogas reactor (1) to said first side of the membrane (20) so as to provide nutrients to the biofilm and for absorbing the methane gas generated by the biofilm of methanogens (21); and
  ▪ a fluid return line (11) in fluid connection with said first side of the membrane for returning the methane enriched fluid from the membrane biofilm reactor (9) to the biogas reactor (1).

A supply line (17, 18) may be arranged for conveying gas, from the biogas reactor (1) to the membrane biofilm reactor (9), said supply line being different from said liquid intake line (6) and said supply line (17, 18) is particularly arranged for conveying gas, to said second side of the membrane (20) in said membrane biofilm reactor (9) and said supply line (17, 18) is in fluid connection with a hydrogen gas ($H_2$) source (15), preferably an external hydrogen gas source, capable of regulating the amount of hydrogen in the supply line (17, 18).

Another aspect of the present invention relates to a method for increasing the methane content of biogas, the method comprising

- generating biogas in a biogas reactor (1); and
- converting carbon dioxide ($CO_2$) in the biogas and supplied hydrogen ($H_2$) into methane ($CH_4$) in a membrane biofilm reactor, the membrane biofilm reactor comprising a biofilm of methanogens (21) on, or in, a membrane (20), said membrane comprising a first side and a second side;

the membrane biofilm reactor (9) being positioned separately and outside of the biogas reactor (1), the biogas reactor being in fluid connection with the membrane biofilm reactor (9) by:

  ▪ a fluid intake line (6) for circulating fluid from the biogas reactor (1) to said first side of the membrane (20) so as to provide nutrients to the biofilm and for absorbing the methane gas generated by the biofilm of methanogens (21); and
  ▪ a fluid return line (11) in fluid connection with said first side of the membrane for returning the methane enriched fluid from the membrane biofilm reactor (9) to the biogas reactor (1).

A supply line (17, 18) may be arranged for conveying gas, from the biogas reactor (1) to the membrane biofilm reactor (9), said supply line being different from said liquid intake line (6) and said supply line (17, 18) is particularly arranged for conveying gas, to said second side of the membrane (20) in said membrane biofilm reactor (9) and said supply line (17, 18) is in fluid connection with a hydrogen gas ($H_2$) source (15), preferably an external hydrogen gas source, capable of regulating the amount of hydrogen in the supply line (17, 18).

Yet another aspect of the present invention is to provide a computer program product being adapted to enable a computer system comprising at least one computer having data storage means in connection therewith arranged for performing the method according to the invention, implemented in a system according to the invention.

**Brief description of the figures**

Figure 1 shows a schematic representation of integrated power to gas and biogas upgrading system.

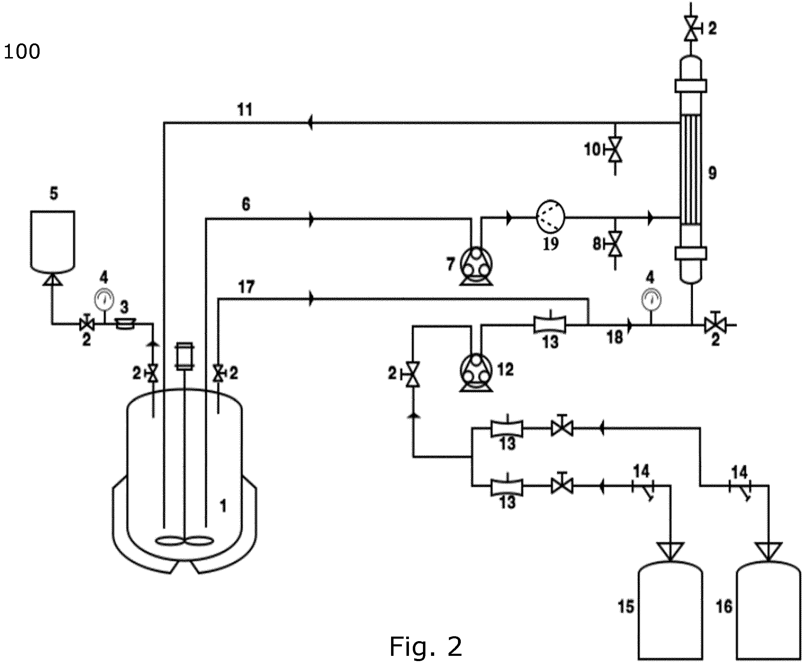

Figure 2 shows a simplified diagram of Membrane biofilm reactor based gas upgrading system. 1) Temperature controlled Continuously Stirred Tank Reactor (CSTR); 2) Valve; 3) Gas sampling septum port; 4) Pressure gauge; 5) Upgraded gas collection bag; 6) Liquid intake line; 7) Liquid recirculation pump; 8) Effluent/sample withdrawal line; 9) Membrane module; 10) Feed liquid injection line; 11) Liquid return line; 12) Gas feed pump; 13) Gas flow meter; 14) Gas valve; 15) Hydrogen gas feed bag; 16) Biogas/Carbon dioxide gas feed bag; 17) Gas recirculation line; 18) Gas feed to lumen side of the membrane; 19) Self cleaning strainer or coarse microfilter of the dead end type with a particle cut-off size of 300 $\mu$m to 100 $\mu$m preferably 100 $\mu$m.

Figure 3 shows a "Reactor I" upgraded biogas composition over the course of operation.

Figure 4 shows a "Reactor II" upgraded biogas composition over the course of operation.

Figure 5 shows profiles of $CH_4$ formation and $H_2$ consumption A) Profile of $CH_4$ formation in Reactors I & II. B) Profile of $H_2$ consumption in Reactors I & II.

Figure 6 shows s close-up schematic figure of a membrane module including drawings of gas diffusion through the membrane to the liquid phase, details of a porous membrane, non-porous membrane and composite membrane.

Figure 7 shows gas delivery to the internal space of the membrane and diffusion through two parallel membrane walls, biofilms and the surrounding liquid.

Figure 8 shows gas delivery to the internal space of the membrane and diffusion through the encircling membrane walls, biofilm retained on the membrane and the surrounding liquid.

Figure 9 shows various gas and liquid delivery configurations to the membrane module a) co-current mode b) counter current mode c) Combination of co current and counter current modes.

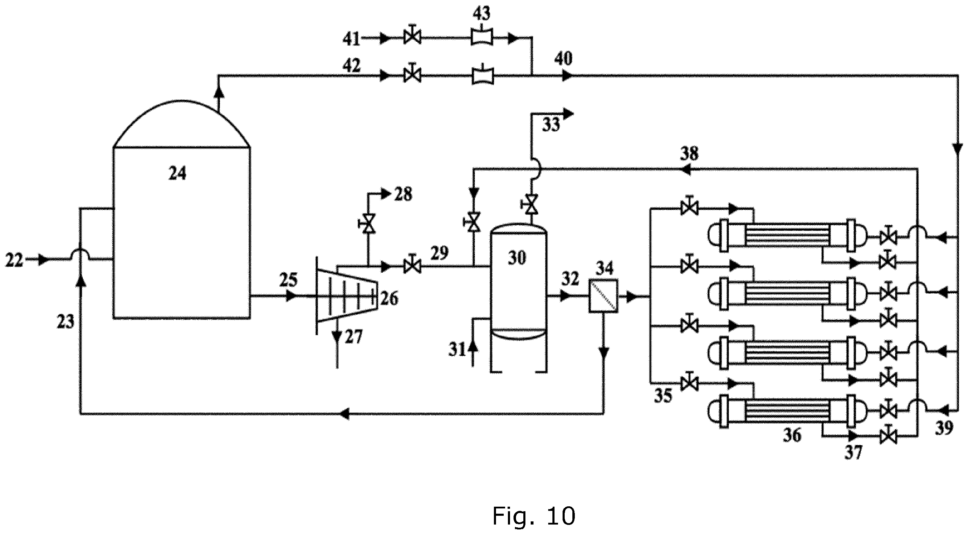

Figure 10 Alternative Membrane biofilm reactor based gas-upgrading system. 22) Biomass feed line to main digester; 23) Return line for separated slurry; 24) Main biogas digester; 25) Digested effluent; 26) Decanter centrifuge; 27) Separated solid; 28) Excess effluent; 29) Feed line to Upgrading digester; 30) upgrading digester; 31) Nutrient feed line; 32) Effluent line; 33) Upgraded natural gas quality biogas; 34) self-cleaning microfilter; 35) Liquid feed to membrane shell side; 36) Bank of membrane modules; 37) Liquid outlet from membrane shell side; 38) Main liquid recirculation line to upgrading digester feed line; 39) Mixed gas feed line to lumen side of the membrane; 40) Main mixed gas line; 41) Hydrogen gas supply line ; 42) Biogas line ; 43) Gas flow meter.

Figure 11 shows principal component analysis (PcoA) of all reactor liquid and biofilm samples.

The present invention will now be described in more detail in the following.

## Detailed description of the invention

### biogas upgrading systems

As mentioned above, an object of the present invention relates to the provision of improved biogas upgrading systems. Thus, as aspect relates to a system (100) for increasing the methane content of biogas, the system comprising

- a biogas reactor (1) capable of generating biogas; and

- a membrane biofilm reactor (9) arranged for converting carbon dioxide ($CO_2$) in the biogas and supplied hydrogen ($H_2$) into methane ($CH_4$), the membrane biofilm reactor being arranged for comprising a biofilm of methanogens (21) on or in, the membrane (20), said membrane comprising a first side and a second side;

wherein the membrane biofilm reactor (9) is positioned separately and outside of the biogas reactor (1), the biogas reactor being in fluid connection with the membrane biofilm reactor (9) by:

  ▪ a fluid intake line (6) for circulating fluid from the biogas reactor (1) to said first side of the membrane (20) so as to provide nutrients to the biofilm and for absorbing the methane gas generated by the biofilm of methanogens (21); and

■ a fluid return line (11) in fluid connection with said first side of the membrane for returning the methane enriched fluid from the membrane biofilm reactor (9) to the biogas reactor (1).

The described system provides several advantages compared to previous biogas upgrading systems. Examples of advantages of the system according to the present invention are:

- Efficient upgrading of the biogas (increased $CH_4$ content). As seen in the example section a high $CH_4$ content is obtained.
- Easy upscaling and repair of the membrane biofilm reactor. Since the membrane biofilm reactor is positioned outside of the main biogas reactor, Thus, easy access is available without having to open the main biogas reactor.
- No need for pre-incubation with optimized micro-organisms. Over time the micro-organisms which thrive the best on the membrane biofilm reactor will dominate in the generated biofilm. Due to the addition of optimized nutrients for methanogens (including $CH_4$ and $CO_2$), these will dominate over time.
- Storage of excess electricity in the form of upgraded biogas can be injected to the natural gas grid;
- At least 90% methane needed for feeding into natural gas supply system, this is achievable with the system according to the invention.

In the present context, the term "fluid intake line" may be used interchangeably with the term "liquid inlet line", or simply "liquid inlet". Similarly, the term "fluid return line" may be used interchangeably with the term "liquid outlet line", or simply "liquid outlet".

In the present context, the term "membrane" relates to a permeable barrier between a gas and a liquid phase to keep the phases separate while promoting conversion of the liquid phase or gas phase components. The membranes could be microporous or non-porous membranes (dense polymeric gas separation membrane) wherein the gas dissolves and diffuses through the dense membrane wall characterized by the ability of the gases CO2 and H2 to permeate the membrane or membranes having similar properties that transfer or dissolve gases into liquids while simultaneously serving as the support upon which the fermenting cells grow as a biofilm in a concentrated layer.

In the present context, the term "biofilm" relates to a film of a microbial community. In a preferred embodiment, the film is an anaerobic microbial communities capable of converting gaseous components $CO_2$ and $H_2$ to Methane. In the present context "comprising a biofilm" relates to the situation where the membrane is loaded with a biofilm, or a biofilm has occupied the pores of the membrane or part of the pores. In the present "Arranged for comprising" context to be understood to be able to allow for the growth of a biofilm.

The system may comprise a further supply line. Thus, in an embodiment, a supply line (17, 18) is arranged for conveying fluid and/or gas, preferably gas, from the biogas reactor (1) to the membrane biofilm reactor (9), said supply line being different from said liquid intake line (6). In yet an embodiment, said supply line (17, 18) is particularly arranged, for conveying fluid and/or gas, preferably gas, to said second side of the membrane (20) in said membrane biofilm reactor (9). In another embodiment, said supply line (17, 18) is in fluid connection with a hydrogen gas ($H_2$) source (15), preferably an external hydrogen gas source, capable of regulating the amount of hydrogen in the supply line (17, 18). In yet another embodiment, the said supply line (17, 18) is in fluid connection with a $CO_2$ source (16) and/or biogas source capable of regulating the amount of $CO_2$ and/or biogas in the supply line.

In a preferred embodiment, a supply line (17, 18) is arranged for conveying gas, from the biogas reactor (1) to the membrane biofilm reactor (9), said supply line being different from said liquid intake line (6) and said supply line (17, 18) is particularly arranged for conveying gas, to said second side of the membrane (20) in said membrane biofilm reactor (9) and said supply line (17, 18) is in fluid connection with a hydrogen gas ($H_2$) source (15), preferably an external hydrogen gas source, capable of regulating the amount of hydrogen in the supply line (17, 18).

In a preferred embodiment, the system does not comprise a membrane biofilm reactor inside a biogas reactor. As mentioned above, an advantage of this, is easy access to the membrane biofilm reactor.

In yet a preferred embodiment, the biofilm is supplied with exogenously supplied H2 outside a biogas reactor.

In yet a preferred embodiment, the systems allows for a circular flow of liquid/ gas between the biogas reactor (1) and the membrane biofilm reactor (9). In this way, a constant flow of e.g. nutrients is provided to biofilm reactor. Another advantage is that the $CH_4$ enriched liquid /gas is returned to the biogas reactor from where it can be collected.

In yet a further preferred embodiment, the system is adapted to allow for repair and/or shift the all or part of the membrane biofilm reactor, without having to access a biogas reactor.

In another preferred embodiment, said supply line (17, 18) is in fluid connection to the gas phase (upper part) of the biogas reactor.

In more specific embodiments (with (non-limiting) reference to figure 10) the effluent (25) from main biogas digester (24) may be screened through a centrifuge decanter (26). The clarified liquid (29) and a recirculating liquid (38) may in an embodiment be jointly fed to an upgrading digester (30) that stores filtered liquid from the biogas reactor containing the microbial strains capable of converting carbon dioxide to methane by reaction with hydrogen. This digester (30) may in an embodiment function both as a buffer tank for the operation of the membrane biofilm reactor (36) and/or as receiver for the upgraded natural gas quality methane produced and withdrawn via line (33). To facilitate the growth of microorganisms in the digester (30), nutrient solution containing macro- and micro-nutrients (such as Nitrogen, Phosphorous, and Sulfur) may in an embodiment be fed through line (31). The effluent (32) from the upgrading digester (30) may in an embodiment be screened by microfilter screen (34) to separate particulate matter that may otherwise block liquid passage inside membrane module (36).

In an embodiment, the liquid may enter the membrane shell side via line (35) and exits via line (37) after close contact with the gas mixture supplied through lumen side of the membrane via line (39). The main Gas mixture (40) supplied to the membrane modules may in an embodiment constitute a stoichiometric mixture of hydrogen gas (41) and biogas (42).

Thus, in yet an embodiment, the fluid intake line (6) for circulating fluid from the biogas reactor (1) to said first side of the membrane (20) is connected via a self-cleaning strainer (19) and/or a coarse microfilter of the dead end type with a particle cut-off size of 300 $\mu$m to 100 $\mu$m preferably around 100 $\mu$m.

In yet a further embodiment, the the fluid intake line (6) for circulating fluid from the biogas reactor (1) to said first side of the membrane (20) is further connected via an upgrading digester (30). In yet an embodiment a microfilter is positioned on each side of the digester (30) (see figure 10).

The membrane in the membrane biofilm reactor (9) may have different characteristics. Thus, in an embodiment, the membrane (20), is a microporous membranes, having an average pore size in the range 0.05 - 1 $\mu$m, such as in the range 0.1 - 0.5 $\mu$m, or such as in the range 0.1- 0.3 $\mu$m, e.g. around 0.2 $\mu$m . In another embodiment, the membrane is hydrophobic. In the example section hydrophobic membranes having an average pore size of around 0.2 $\mu$m is used. An advantage of the hydrophobic membrane is that fluid does not easily transfer from the (outer) fluid side to the (inner) gas side. In another embodiment, the membrane is a dense non-porous membrane permeable to the gases $CO_2$ and $H_2$.

Different types of membrane may also be used. Thus, in an embodiment, the membrane (20) is selected from the group consisting of a tubular membrane, a capillary membrane, a hollow fiber membrane and a flat sheet membrane. The membrane material may also differ. Thus, in an embodiment, the membrane material is selected from the group consisting of either porous membranes: Polypropylene (PP), polyethylene (PE), polyvinylidene fluoride (PVDF), and polyvinyl chloride (PVC) or other polymeric dense (non-porous) membranes like polyimide, polysulphone, or cellulose acetate, preferably polysulphone. In yet an embodiment, the membrane is a plurality of capillary membranes arranged in one, or more, common array, such as 5 - 100 membranes such as 10 - 80 membranes or such as 30-60 membranes.

A purpose of the membrane is to promote the generation of a biofilm in or on the membrane. Thus, in an embodiment, the membrane (20) on the first side comprises a biofilm attachment promoting surface. Such biofilm attachment promoting surfaces may be provided of different types. Thus, in an embodiment the membrane (20) on the first side is covered with webbing, such as braided nylon webbing, and/or is coated with a chemical, such as organosilane, and/or the surface is modified, such as by etching, for promoting biofilm attachment.

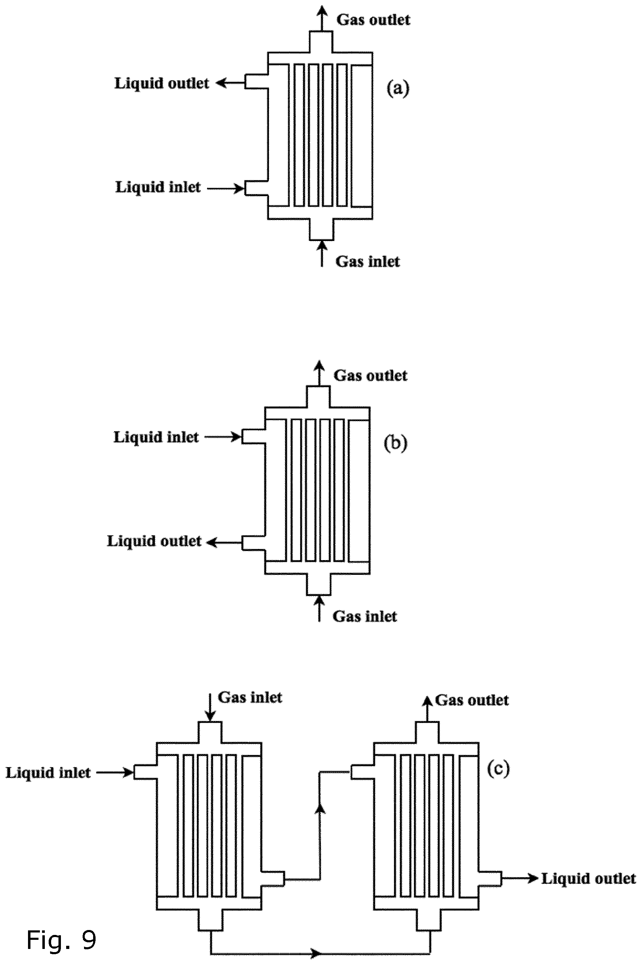

Depending on the size of the biogas reactor and/or the size of the biofilm membrane modules (9), the number of biofilm membrane modules (9) in the system may vary. Thus, in another embodiment, the system comprises multiple biofilm membrane modules (9). Such multiple biofilm membrane modules (9), may be arranged in different formats. Thus, in yet a further embodiment, the multiple membrane modules are arranged for being operated in co-current mode (see Figure 9A), counter-current mode (see Figure 9B), or a combination thereof (see Figure 9C) between the gas and the liquid.

Different types of membrane biofilm reactors exists. Thus, in an embodiment the membrane biofilm reactor is a hollow-fiber membrane biofilm reactor or a membrane biofilm gas-liquid contactor, preferably a hollow-fiber membrane biofilm reactor.

The type of methanogens which may occupy the membrane (preferably in the form of a biofilm may vary. Thus, in an embodiment, the methanogens are hydrolytic, acidogenic, acetogenic and/or methanogenic microorganisms. In yet an embodiment, the methanogens are selected from the group consisting of hydrogenotropic methanogens that comprises one or more hydrogenotrophic methanogenic archaea species selected from the group consisting of Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum,Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermautotrophicum, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barken, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus, and Methanopyrus kandleri.

As mention above, the methanogens may origin from the bacteria naturally present in the inoculum of the biogas reactor. These methanogens may then be circulated to the membrane biofilm reactor through the fluid intake line (6), where they may settle on the membrane. It is noted that in a certain embodiment the membrane is preloaded with methanogens before being coupled to the biogas reactor.

In an embodiment, the membrane biofilm reactor comprises a biofilm (21) of methanogens on or in, the membrane. In yet an embodiment, the biofilm comprises at least 90% by number of hydrogenotrophic methanogens, such as at least 95%, such as at least 98% or such as at least 99%.

In the system according to the invention, the initial biogas is generated in the biogas reactor. Thus, in an embodiment the biogas reactor comprises a liquid or semiliquid digestate and a gas phase comprising biogas. In yet an embodiment, the digestate comprises components selected from the group consisting of the effluent after anaerobic digestion/co digestion of crops, such as maize, silage, sewage, sludge, manure, and/or food waste, stillage, municipal organic waste and green waste.

Biogas upgraded in the system according to the invention should of course also be collected at some stage. Thus, in an embodiment the system further comprises one or more collection units (or collection line). The collection unit may be positioned at different locations in the system. Thus, in an embodiment the collection unit is connected to the biogas reactor (preferably upper part), to the membrane biofilm reactor, and/or to the fluid return line (11), preferably to the biogas reactor. In another embodiment, upgraded biogas is collected from the upgrading digester or buffer tank (30). The one or more collection units may be storage units, or transfer units for directly transferring the upgraded biogas to the natural gas grid.

*Method for increasing the methane content of biogas*

The present invention also relates to a method for increasing the methane content of biogas. Thus, in an aspect the invention relates to a method for increasing the methane content of biogas, the method comprising

- generating biogas in a biogas reactor (1); and
- converting carbon dioxide ($CO_2$) in the biogas and supplied hydrogen ($H_2$) into methane ($CH_4$) in a membrane biofilm reactor, the membrane biofilm reactor comprising a biofilm of methanogens (21) on or in, a membrane (20), said membrane comprising a first side and a second side;

the membrane biofilm reactor (9) being positioned separately and outside of the biogas reactor (1), the biogas reactor being in fluid connection with the membrane biofilm reactor (9) by:

 ▪ a fluid intake line (6) for circulating fluid from the biogas reactor (1) to said first side of the membrane (20) so as to provide nutrients to the biofilm and for absorbing the methane gas generated by the biofilm of methanogens (21); and

 ▪ a fluid return line (11) in fluid connection with said first side of the membrane for returning the methane enriched fluid from the membrane biofilm reactor (9) to the biogas reactor (1);

In an embodiment, a supply line (17, 18) is arranged for conveying fluid and/or gas, preferably gas, from the biogas reactor (1) to the membrane biofilm reactor (9), said supply line being different from said liquid intake line (6). In yet an embodiment, said supply line (17) is particularly arranged, for conveying fluid and/or gas, preferably gas, to said second side of the membrane (20) in said membrane biofilm reactor (9). In another embodiment, said supply line (17,18) is in fluid connection with a hydrogen gas ($H_2$) source (15), preferably an external hydrogen gas source, capable of regulating the amount of hydrogen in the supply line (17, 18). In yet another embodiment, the said supply line (17, 18) is in fluid connection with a $CO_2$ source (16) and/or biogas source capable of regulating the amount of $CO_2$ and/or biogas in the supply line.

In a preferred embodiment, a supply line (17, 18) is arranged for conveying gas, from the biogas reactor (1) to the membrane biofilm reactor (9), said supply line being different from said liquid intake line (6) and said supply line (17, 18) is particularly arranged for conveying gas, to said second side of the membrane (20) in said membrane biofilm reactor (9) and said supply line (17,18) is in fluid connection with a hydrogen gas ($H_2$) source (15), preferably an external hydrogen gas source, capable of regulating the amount of hydrogen in the supply line (17, 18).

In an additional embodiment, the method further comprises collecting the upgraded biogas.

In another preferred embodiment, said supply line (17, 18) is in fluid connection to the gas phase (upper part) of the biogas reactor.

To optimize the conversion rate to methane, the provided gas to the membrane bioreactor should also be optimized. Thus, in an embodiment, the method is adapted for providing a gas to the second side of the membrane, having a molar ratio of $H_2$ to $CO_2$ in the range 6:1 to 3.5:1, such as 5:1 to 3.5:1 or such as about 4:1 molar ratio of $H_2$ to $CO_2$. As described above, theoretically a ratio of 4:1 should be optimal.

It is of course preferred that fluid from the "fluid side" of the membrane does not leak to the "gas side" of the membrane. Thus, in an embodiment, the pressure on gas side (lumen side) of the membrane is higher than the pressure on the liquid side (shell side) of the membrane. In another embodiment, the pressure difference between the gas and liquid sides is in the range of 0.05 to 15 bar, preferably 0.5 to 6 bar, and most preferably 1 to 3 bar (with the highest pressure on the gas side).

Methanogens are anaerobic organisms. Thus, in an embodiment the biogas reactor is kept under anaerobic conditions. In yet an embodiment, the biogas reactor is operated under substantially constant stirring.

As previously mentioned, $H_2$ is used in the method (and system) according to the invention. In an embodiment, the provided $H_2$ has been generated by electrolysis. In yet an embodiment, the power for the electrolysis is supplied from a renewable energy source, such as wind power or solar power.

The upgraded biogas collected, should preferably have a high content of methane and low content of $H_2$ and $CO_2$. Thus, in an embodiment, collected upgraded biogas has a methane content above 95% by volume, such as above 97%, such as above 98% such as above 99%, or such as above 99.5%. Example 3 shows that a level of methane of 99.1% may be reached.

In yet an embodiment the amount of $H_2$ in collected biogas is below 2.2% by volume such as below 2%, preferably below 1.5%, more preferably below 1%, such as below 0.8%. In example 3 $H_2$ levels in upgraded gas is as low as 0.6%.

In yet an embodiment the amount of $CO_2$ in collected biogas is below 2.0% by volume, such as below 1.5%, preferably below 1.0%, more preferably below 0.5%, such as below 0.3%. In example 3, low levels of $CO_2$ are documented.

Without being bound by theory it is believed that by having the bacteria positioned directly on/in the membrane as a biofilm (and separated from the bacteria generating the methane in the bioreactor (inoculum)), there will be a selection of bacteria in the biofilm, which are optimized for utilizing the available $CO_2$ and $H_2$ optimally. This is underlined in example 3, which shows that the bacteria in the inoculum and in the biofilm are different populations, with a prevalence of the methano-bacteria in the biofilm population.

In yet an embodiment, the system operates/is operable at a pH at above 8.4, such as above 8.6, such as above 8.8 or such as in the range 8.4-8.9 or 8.6 - 8.9. The bioreactor system according to the invention efficiently operates under high pH conditions.

In a similar embodiment, the pH in the first bioreactor (e.g. in the inoculum) is above 8.4, such as above 8.6, such as above 8.8, or such as in the range 8.4-8.9, in the range 8.5-8.9 or in the range 8.6 - 8.9. As presented in example 3, the average pH in the bioreactors is in the range of 8.4-8.90. This indicates that the present invention is robust and accommodates possible processing instabilities that may lead to significant pH shifts, e.g. pH's above 8.4.

*Computer program product*

In yet an aspect, the invention relates to a computer program product being adapted to enable a computer system comprising at least one computer having data storage means in connection therewith arranged for performing the method according to the invention, implemented in a system according to the invention.

It should be noted that embodiments and features described in the context of one of the aspects of the present invention also apply to the other aspects of the invention. Especially the features/embodiments of the system aspect may also apply to the method and computer program aspects.

All patent and non-patent references cited in the present application, are hereby incorporated by reference in their entirety.

The invention will now be described in further details in the following non-limiting examples.

**Examples**

**Example 1**

Conversion system according to the invention

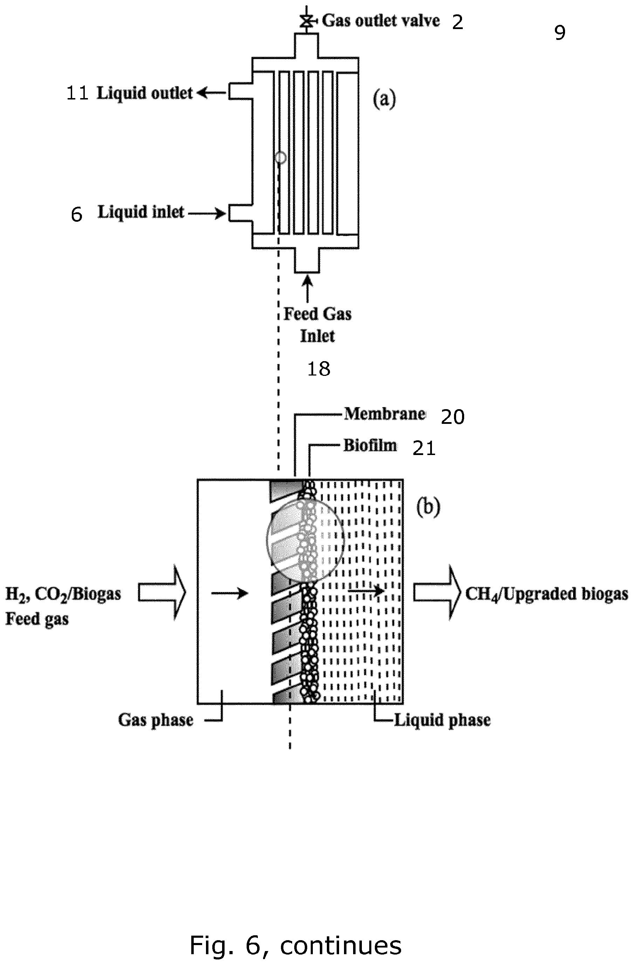

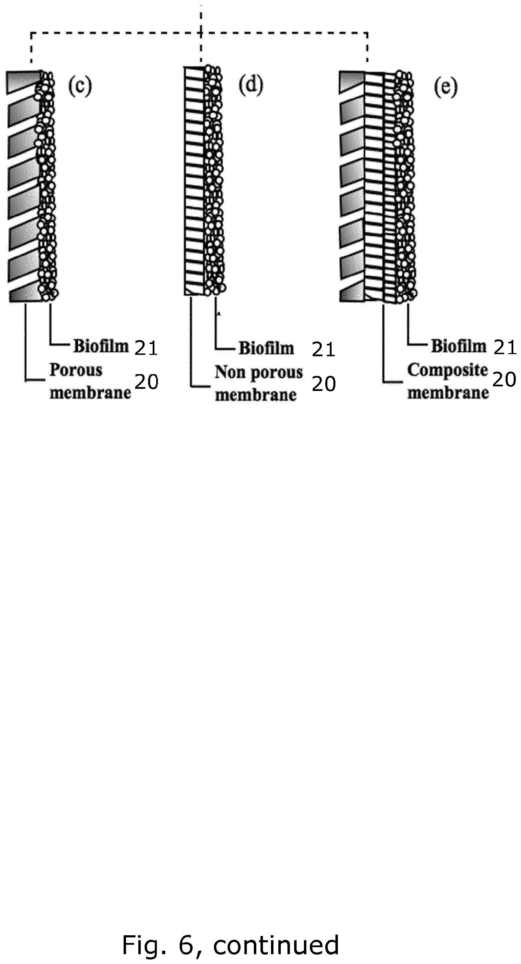

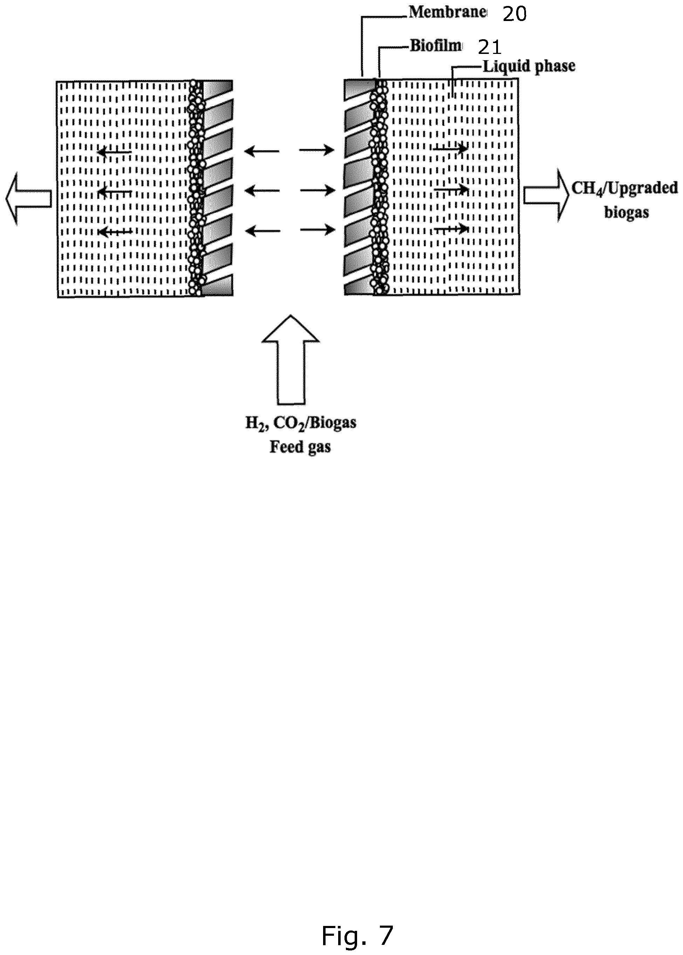

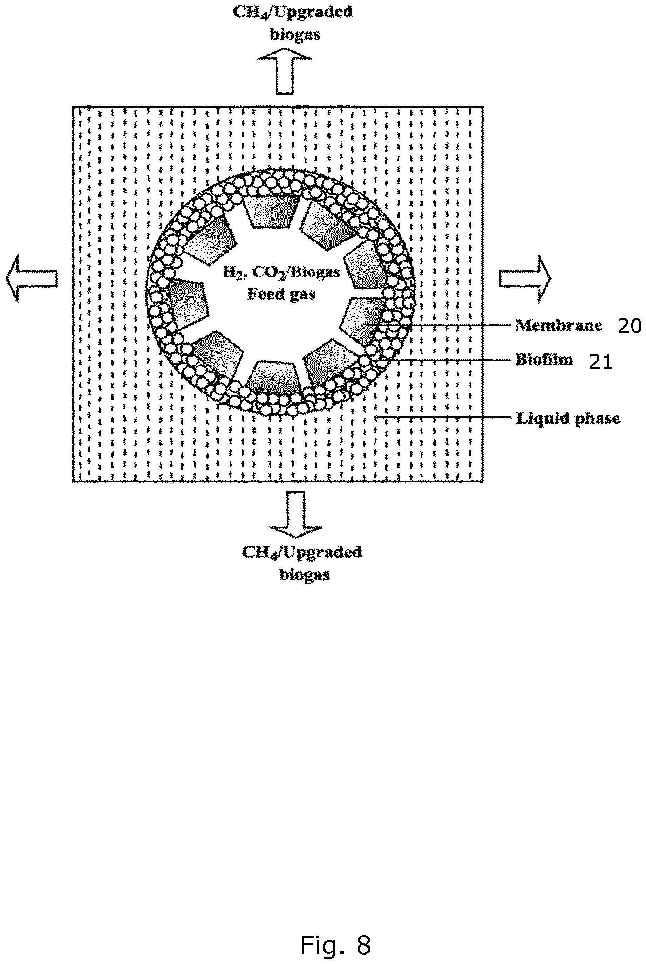

Pumping gaseous substrates through the membrane lumen (internal space) of the membrane biofilm reactor 9 allows the gas to diffuse through gas filled micropores into the biofilm, where it is transformed to methane by the microbes. Furthermore, the membrane biofilm reactor liquid can be continuously recirculated in the shell side of the membranes using a pumping system. The recirculation facilitates the delivery of essential micro- and macro-nutrients required for microorganisms in the biofilm; it also homogenizes the environmental conditions like pH and temperature. The recirculation rate also serves to control the biofilm thickness maintained over the membrane surface. Similarly, the product gas (methane) formed in the biofilms will be transported by the recirculating liquid to the reactor tank where it will disengage to the gas phase due to extremely low solubility of methane. Finally, the pressure gradient in the reactor headspace will push out the methane to the gas collection unit 5. Schematic representation of the membrane biofilm reactor 9 coupled to continuous stirred tank biogas reactor 1 is shown in Figure 2. Figures showing examples of membrane biofilm bioreactors 9 are shown in figures 6-9.

The core of this process is a group of hydrogenotrophic methanogens, which are naturally present as part of a larger group of anaerobic microbial communities such as hydrolytic, acidogenic, acetogenic and methanogenic micro-organisms. Hydrogenotrophs grow feeding on $CO_2$ and $H_2$ while producing methane in the process. Therefore, the exclusive supply of these gaseous substrates via the membranes enhances the growth and relative abundance of hydrogenotrophs within the biofilm and reactor liquid. In addition, the microbes require various macro- and micro-nutrients for growth and metabolism. The biogas reactor liquid is usually a digestate containing the inoculum and nutrients necessary for the start-up of the anaerobic digestion process; however, the liquid could also be based entirely on nutrient solution.

The present invention uses microporous membranes as the support upon which the microbial cells attach to as a biofilm and are thus immobilized as stratum. The result is a highly efficient and economical transfer of the gaseous components at nearly 100% utilization (see also example 3 and corresponding figures), overcoming the limitations of other gas liquid mass transfer methods employed in a bioreactor configurations such as gas lift reactors.

Hydrophobic microporous membranes are preferable for this application. Furthermore, it is necessary to properly manage the pressure difference across the membrane to avoid membrane wetting and subsequent decline in the gas transfer rate.

**Example 2**

Example of configuration of system according to the invention

Figure 2 illustrates a specific configuration of one embodiment of this invention. A gas supply line 18 delivers a feed gas constituting a stoichiometric mixture of $H_2$ and $CO_2$ or biogas at a rate recorded by gas flow meter 13. Another embodiment could also include gas recycle line 17 that joins the feed stream 18 after gas flow meter 13. The membrane biofilm reactor 9 receives the feed gas stream to the lumens of the membranes 19. As the biofilm reactor is operated with fully closed gas outlet valve, the gas will fill membrane pores and diffuses to the biofilm layer 20 maintained over the membrane exterior. An alternate variant configuration could be multiple membrane biofilm reactors 9 operated multi-stage using the biofilm reactors in counter-current, co-current or a combination thereof mode between the gas and the liquid (see figure 9A-C). Liquid exits the membrane biofilm reactor through return line 11 to the continuously stirred biogas reactor 1. The biogas reactor provides the means of temperature and pH controls for the liquid, which contains nutrients needed to sustain the activity of the microbial cells. The liquid in the biogas reactor may be stirred to provide adequate mixing and homogenization. A nutrient feed may be added via line 8, as needed, to replenish nutrients. Similarly, effluent may be withdrawn via line 10, as needed, to maintain constant volume and remove extra water produced from the biochemical reaction. However, the system would not be prone to washout since the hydraulic retention time could be kept high, since feeding substrates are gaseous. The flow rate of stream 6, recirculated through the membrane biofilm reactor, is regulated so that there is no significant liquid boundary layer that increase resistance to mass transfer near the liquid side of the membrane and there is no excessive shear that may severely limit the attachment of cells and formation of the biofilm on the membrane surface.

**Example 3**

*Aim of study*

Verifying the efficiency of the bioreactors according to the invention.

*Materials and Methods*

Inocula

The starting material was an acclimatized thermophilic inoculum from a UASB reactor (Upflow Anaerobic Sludge Blanket reactor) that has been running in a laboratory for six months. The inoculum was rich in hydrogenotrophic methanogen bacterial community since the reactor was exclusively fed with gaseous mixture ($H_2$ & $CO_2$ in the ratio of 4:1) for the whole of its operation. As a source of nutrients, thermophilically digested manure (Biogas Plant, Snertinge, Denmark) were collected and kept in 55°C controlled incubator for one month before use. The storage was intended to ensure degassing and exhausting of the available biodegradable carbon sources.

*Experimental setup*

Two sets of membrane bioreactors (MBRs) were run in parallel. Both setups constituted a 2L volume stirred vessels coupled to an externally installed polymeric membrane modules. Commercial modules (Microdyn®) made of a hydrophobic microporous polypropylene membranes were used as an interface between the gas and liquid phases and as a support for the microbial population. The first module (Reactor I) comprised a bundle/plurality of 40 capillary membranes of 0.2 $\mu$m nominal pore size and 500 mm length. The inside and outside diameters of the fibers were 1.8 mm and 2.2 mm respectively, the total active area was 0.138 m$^2$. The second module (Reactor II) was a single strand tubular membrane with 0.2 $\mu$m pore size, 5.5 mm ID, 8.0 mm OD and 500 mm length; this membrane was fixed inside a metallic membrane housing (MI-10x500-PN25-TC-02). Total surface area of the membrane was 0.0125 m$^2$. Moreover, the external surface of this membrane was covered in braided nylon webbing that was expected to promote biofilm attachment. Temperature of the system was maintained in the thermophilic region (55°c) using a heating plate and a thermally regulated heating tape fixed over the body of the stirred tanks. Besides, the vessels were wrapped with an insulation padding to minimize heat dissipation.

The reactors were stirred 200-450 rpm using magnetic stirrer to ensure adequate mixing. Further homogenization was achieved by continuously recirculating the broth using separate peristaltic pumps (*WATSON MARLOW* model 120s) between the tanks and shell side of the membrane modules via the lateral ports. Liquid feeding and effluent withdrawal was done manually using syringes via side stream valves. The same side streams served for introducing 0.1M HCl solution for pH adjustment. The supply of gaseous substrate to the lumen side of the membrane was administered using a multichannel peristaltic pump (*OLE DICH, DENMARK*). The membranes were operated in a dead end configuration, the gaseous mixture constituted 62% H$_2$, 23% CH$_4$ and 15% CO$_2$, it was supplied in pressurized cylinders by AGA, Denmark. However, daily a specified volume of the gas mixture was transferred in to a gas tight bag and the reactors were fed from these bags. The upgraded biogas from the reactors was collected in a separate gas tight bags and the volume was daily measured by connecting the bags to a graduated water displacement gas meter column. Similarly, the volume of unconsumed gas mixture remaining in the feed bags was measured. The net gas consumption was calculated by deducting the gas volume remaining from the capacity of the feed bag. Schematic depiction of the bioreactor configuration is shown in Figure 2.

*Analytical methods*

Total solids (TS), Volatile Solids (VS), and Total Kjeldahl Nitrogen (TKN) were analysed according to APHA. Volatile fatty acids were determined by a gas chromatograph (GC-2010; Shimadzu) with a flame ionization detector and FFAP column, the detection limits for VFA analysis were 5-1500mg/L. H$_2$ was analyzed by GC-TCD fitted with a 4.5 m$\times$3 mms-m stainless column packed with Molsieve SA (10/80). CH$_4$ was analyzed with a gas chromatograph equipped with a TCD fitted with paralleled column of 1.1 m$\times$3/16" Molsieve 137 and 0.7 m$\times$1/4" chromosorb 108. Standard gas mixtures of CH$_4$, CO$_2$ and H$_2$ were utilized to determine calibration curves (5-100%).

Genomic DNA extraction and sequencing

Liquid samples of 50ml volume were collected in triplicates (identified as a, b & c) from each reactor at the end of phase IV and just after completion of the whole experiment (phase V). Similarly, biofilm samples were also collected from each membrane module (from the biofilm reactor) after the end of the experiment (phase V). PowerSoil DNA Isolation Kit (MO BIO laboratories Inc., Carlsbad, CA USA) was used for genomic DNA extraction and purification; furthermore, a sample quality improvement protocol was followed including an extra purification step using Phe:Chl:IAA. Quality and quantity of the genomic DNA were determined using NanoDrop (ThermoFisher Scientific, Waltham, MA) and agarose gel electrophoresis. The hypervariable V4 region of bacterial and archaeal 16S rRNA genes was amplified using universal primers 515F/806R and sequenced with the MiSeq platform (Illumina). PCR amplification, quality controls and sequencing were performed at the Ramaciotti Centre for Genomics (Sydney, Australia).

Analysis of the 16S rRNA gene sequences

Bioinformatic analyses on raw data were performed using CLC Workbench software (V.8.0.2) with Microbial genomics module plug in (QIAGEN). Quality and chimera crossover filtering were performed using default parameters. Operational taxonomic units (OTUs) clustering and taxonomic assignment were done using Greengenes v13_5 database as reference (clustered at 97%) with multiple sequence comparison by log-expectation on fixed length trimmed (240bp) sequences. New OTUs were indicated when similarity percentage was lower than 80% with minimum occurrence of five reads. Low abundant OTUs were discarded from further analyses (less 10 reads, less 0.01% relative abundance). MUSCLE software (Edgar, 2004) was used for sequence alignment in order to create a maximum likelihood phylogeny with neighbor joining as construction method and Jukes Cantor as nucleotide substitution model and 100 bootstrap replicates. Alpha diversity was calculated using number of OTUs, Chao 1 bias-corrected and Phylogenetic diversity; Beta diversity was obtained using Bray-Curtis matrix and represented as Principal Coordinate Analysis (PCoA). The study focused on the high abundant OTUs (i.e. relative abundance higher than 0.5% with respect to the total number of sequences) which were present in at least one upgrading reactor.

*Bioreactors start-up*

The reactors were started up with 400 mL of inoculum and 1200 mL digested manure. Both reactors and corresponding membrane configuration were tightly sealed then flushed with nitrogen gas to eliminate any residual oxygen present in the system. Daily the reactors were fed with 55ml of digested manure as a source of macro- and micro-nutrients; however the carbon source was the carbon dioxide present in the gaseous feed mixture (62% $H_2$, 23% $CH_4$ and 15% $CO_2$). Throughout the reactors operation attempts to adjust pH were targeted to regulate excessive pH increase, otherwise the reactors pH was self-regulated.

*Methanogenic activity tests*

Methanogenic activity test samples were taken from both reactors at three different phases of operation. The test compared methanation potential of the reactors broth by feeding gaseous mixture $H_2$ and $CO_2$ as substrates. 10 ml reactor liquid was directly transferred to 36 ml serum bottles, sealed with a rubber (Rubber B.V., Hilversum, NL) and aluminum caps (Wheaton, Millville, NJ). Then, the samples were immediately supplemented with 26 ml H2/CO2 (80:20, 1 atm) as substrates. Bottles with reactor samples only (without addition of gaseous substrates) were used as controls. The bottles were incubated on a shaker at 55 °C with shaking speed of 300 rpm. All the tests were prepared in triplicates. Every 2 hours gas samples were taken from the head space of the serum bottles and analyzed for CH4, CO2 and H2 content.

*Results*

Biogas upgrading performance of the reactors

Phase I

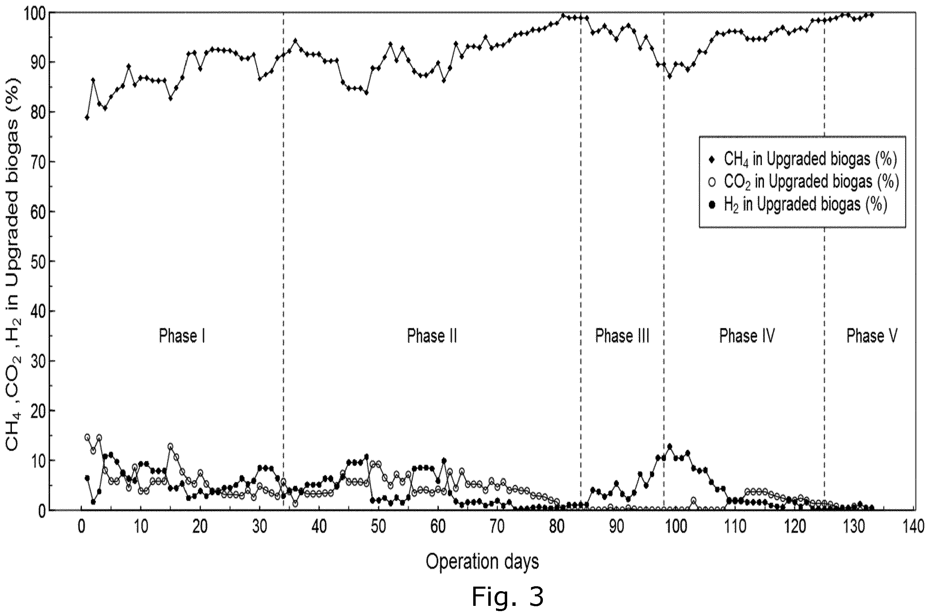

Operation of the reactors was started by maintaining daily gaseous mixture feed rate of 1.76 L/d throughout phase I (figures 3-4). This flow rate corresponded to average gas retention time of 31 hr and daily gas loading rate of 0.77 $L/L_R/d$. Even though both reactors commenced the upgrading from early days of operation, the performance was not steady throughout this initial phase. However, the methane content of upgraded gas has shown gradual increment. By the end of phase I, reactor I and II achieved 87.2% and 85.3% $CH_4$ in upgraded gas respectively. During this phase, gas recirculation from head space of the reactors to the lumen side of the membranes was continuously done via line 17. Moreover, the digester contents were kept under constant agitation at 200 rpm to ensure gentle mixing and homogenous substrate distribution.

Phase II

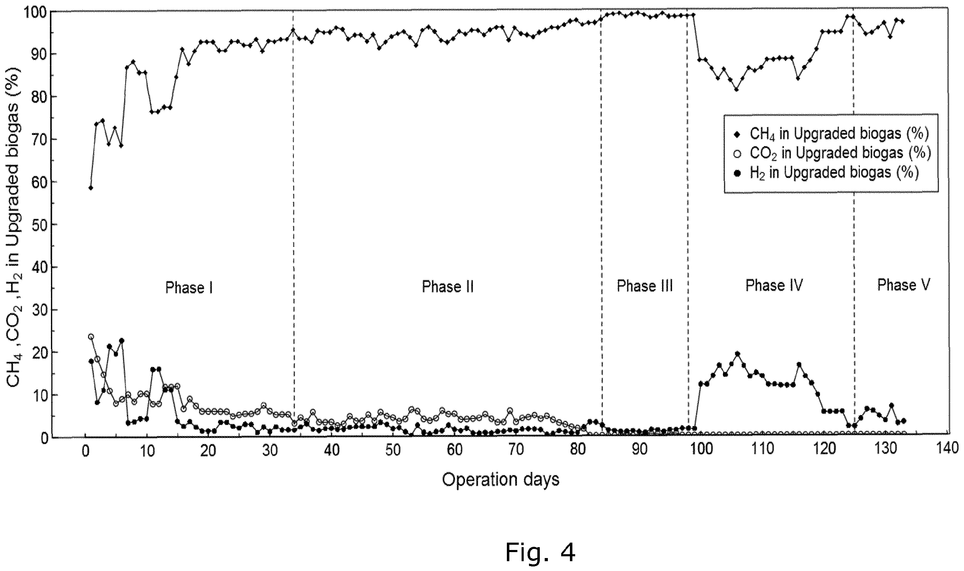

At day 34, the gas loading rate was increased to 1.0 $L/L_R/d$ which correspond to a gas retention time of 23.6 hr. Phase II maintained a higher agitation rate of 350 rpm in an attempt to promote gas liquid mass transfer and subsequent biochemical reaction. Compared to reactor I, reactor II maintained a relatively steady gas upgrading throughout this phase as depicted in figures 3 and 4. In fact, reactor II achieved stable operation after 1 hydraulic retention time while reactor II demanded double hydraulic residence time. Nevertheless, the performance of Reactor II in terms of the level of residual $H_2$ in the upgraded gas was higher than the corresponding residual $CO_2$ throughout this phase. Similarly, Reactor I also manifested this phenomena at later stages of phase 2. Feed gas mixture ratio $H_2/CO_2$ is 4.1, which contain slight extra hydrogen than the stoichiometric requirement. However, the results indicated the presence of more unreacted residual $CO_2$ than $H_2$. Therefore starting from day 70, calculated amount of $H_2$ gas was injected into the gas supply bags using gas tight syringe to marginally raise $H_2/CO_2$ ratio in order to enhance conversion of residual $CO_2$ in the product gas. Besides, gas recirculation was completely stopped as the relative higher proportion of methane in the recirculation gas may lead to drop in the partial pressures of $CO_2$ and $H_2$ in the combined feed stream hampering the ensuing gas liquid mass transfer. After these adjustments, the $CH_4$ content of upgraded gas from both reactors kept steady surge to as high as 99% in Reactor I (RI) and 97% in reactor II (RII). On the other hand, residual $CO_2$ in the upgraded gas declined below detection limits. However, the reactors maintained mean $CH_4$ content of 92% & 94.6% in phase 2.

Phase III and IV

The operations in phases III and IV exhibited a contrasting performance between the two reactors. Phase III commenced with gas loading rate of 1.83 $L/L_R/d$ that lead to drop in gas retention time to 13 hr. The extra hydrogen gas injection intervention instigated in late stages of phase 2 was stopped due to a residual hydrogen increasing tendency in reactor I.
Reactor I showed a steady decline in gas upgrading performance throughout phase III, there was a clear rise in the level of unreacted residual $H_2$ and drop in $CH_4$ content from 98% to 89.5%, this trend could be attributed to a relatively slower adaptation of microbial community in the reactor and membrane module to the loading rate increment.
On the other hand, reactor II maintained peak performance throughout this period, the mean methane content stayed around 98.6% with near complete utilization of reactant species $H_2$ and $CO_2$. Further increase in gaseous substrate loading during Phase IV to 3.7 $L/L_R/d$ resulted in deep in the performance of reactor II which otherwise stayed relatively stable at lower loading rates. In contrast, reactor I regained stability and maintained a steady surge in upgrading performance throughout this phase. Despite decline in reactor II upgrading performance and corresponding rise in residual $H_2$ content, the $CO_2$ content in the upgraded gas remained very low. A similar phenomenon was also noticed in reactor I. At later stages of Phase IV, reactor II showed clear signs of performance recovery; as a result, the $CH_4$ content of upgraded gas increased from 81.3% to 97.9% while the mean achievement stayed around 88%.

Phase V

From figures 3 and 4 it can be noted that both reactors were stabilized by the end of Phase IV; hence, the feed gas loading was increased further to 4.61[$L/L_R/d$] in phase V, which translates to a gas retention time of 5 hours. Reactor I maintained the upgrading momentum throughout this phase, which indicates the microbial communities both in the digester and membrane module are clearly adapted to the feed substrates as well as the environment established within the reactor system. Subsequently, the upgraded gas consistently attained $CH_4$ content around 99% and the remnant components constituted below 1%. Similarly, reactor II maintained a moderately steady performance compared to its results in phase IV, despite the extra loading rate in phase V. As a result, the product gas methane composition fluctuated within narrow range (93.9%-96.8%); however, there was still appreciable level of residual hydrogen that ranged between 2.79-6.72%.

Table 1

| Phase | Phase 1 | | Phase 2 | |
|---|---|---|---|---|
| Reactor | RI | RII | RI | RII |
| Stirrer speed [rpm] | 200 | 200 | 350 | 350 |
| Mean daily gas fed rate [L/d] | 1.76 | | 2.33 | |
| Mean daily gas loading rate [$L/L_R/d$] | 0.77 | | 1.0 | |
| Gas Retention time[hr] | 31 | | 23.6 | |
| Gas recirculation rate [L/d] | 1.76 | | 2.33* | |
| pH | 8.64+0.1 | 8.59±0.1 | 8.62+0.1 | 8.70+0.1 |
| Biogas production rate [$L/L_R/d$] | 0.39±0.04 | 0.41±0.05 | 0.41±0.06 | 0.42±0.07 |
| Gas composition [%] | | | | |
| $CH_4$ | 87.2+3.7 | 85.3+9.3 | 92.0+4.1 | 94.6+1.5 |
| $CO_2$ | 6.2±3.3 | 8.4±4.2 | 4.4±2.2 | 3.8±1.4 |
| $H_2$ | 6.0±2.5 | 6.3±5.5 | 3.6±3.2 | 1.6±0.8 |
| Specific $CH_4$ production rate [$L/L_R/d$] | 0.147±0.04 | 0.15+0.03 | 0.14+0.03 | 0.15+0.04 |
| Specific $H_2$ utilization rate [$L/L_R/d$] | 0.60+0.005 | 0.59±0.02 | 0.67±0.08 | 0.68±0.08 |
| Yield [$CH_4/H_2$] | 0.25+0.06 | 0.26+0.06 | 0.21+0.04 | 0.22+0.05 |

Table 1 continued

| Phase | Phase 3 | | Phase 4 | | Phase 5 | |
|---|---|---|---|---|---|---|
| Reactor | RI | RII | RI | RII | RI | RII |
| Stirrer speed [rpm] | 400 | 400 | 450 | 450 | 450 | 450 |
| Mean daily gas fed rate [L/d] | 4.22 | | 8.5 | | 10.6 | |
| Mean daily gas loading rate [L/L$_R$/d] | 1.83 | | 3.7 | | 4.61 | |
| Gas Retention time[hr] | 13 | | 6.4 | | 5 | |
| Gas recirculation rate [L/d] | - | | - | | - | |
| pH | 8.90±0.1 | 8.98±0.1 | 8.40±0.3 | 8.70±0.2 | 8.68±0.1 | 8.55±0.03 |
| Biogas production rate [L/L$_R$/d] | 0.75±0.13 | 0.69±0.09 | 1.55±0.24 | 1.52±0.22 | 1.69±0.04 | 1.91±0.04 |
| Gas composition [%] | | | | | | |
| CH$_4$ | 94.9±2.8 | 98.6±0.4 | 94.5±3.1 | 88.8±4.9 | 99.1±0.4 | 95.5±1.5 |
| CO$_2$ | 0.1±0.06 | 0.2±0.18 | 1.6±1.3 | - | 0.33±0.3 | - |
| H$_2$ | 5±2.9 | 1.2±0.3 | 3.9±3.6 | 11.2±4.9 | 0.6±0.3 | 4.5±1.5 |
| Specific CH$_4$ production rate [L/L$_R$/d] | 0.31±0.12 | 0.27±0.1 | 0.66±0.22 | 0.59±0.20 | 0.62±0.04 | 0.81±0.04 |
| Specific H$_2$ utilization rate [L/L$_R$/d] | 1.12±0.02 | 1.1±0.002 | 2.26±0.04 | 2.22±0.04 | 2.8±0.002 | 12.8±0.001 |
| Yield [CH$_4$/H$_2$] | 0.27±0.11 | 0.24±0.08 | 0.29±0.1 | 0.27±0.09 | 0.22±0.01 | 0.29±0.01 |

As can be noted in table 1, throughout the reactors operation the pH remained relatively higher in the range of 8.4-8.9. In fact, the pH control intervention was done moderately just to avoid excessive increase; otherwise, the process stabilized itself and the microbial communities efficiently operated under high pH conditions. This is underlined in phase V Performance of the reactors where upgrading to methane content of 99.1 and 95.5% was possible in rectors I & II respectively while the reactors liquor pH was 8.68 and 8.55.

Methanogenic Activity test

The Methanogenic activity test served to study microbial kinetics and activity of the reactors' broth at different stages. The 1$^{st}$ test was done at later stages of phase II while the others were from Phase III and IV respectively. Since the samples were directly withdrawn from the reactors under steady operation, a hydrogenotrophic methanogenic activity that is capable of fermenting $H_2$ and $CO_2$ to methane according to Eq.1 was expected to be the predominant metabolic pathway. However, there are also other microbial communities that can catalyze the same gaseous substrates to acetate as shown in Eq.2. The acetate produced by the acetogens could further be biochemically cleaved to methane as shown in Eq3. Although there are different microbial pathways, methane is the main product formed via the hydrogenotrophic methanogenesis as well as the alternate syntrophic pathways.

$$2CO_2 + 4\,H_2 \rightarrow CH_3\,COO^- + H^+ + 2H_2O \qquad (2)$$

$$CH_3COO^- + H_2O \rightarrow CH_4 + HCO_3^- \qquad (3)$$

The kinetics of $H_2$ consumption and subsequent methane formation was studied over a course of 24 hours. The methane production rates during this period exhibited the same trend as bacterial growth curve; hence, the data were fitted to the modified Gompertz equation as described by [11]. Similarly, the $H_2$ consumption rate followed the bacterial thermal inactivation behavior and it can also be fitted to decaying form of reparametrized Gompertz equation as described by [12, 13].

$$y = A\,exp\left\{-exp\left[\frac{\mu_m e}{A}(\lambda - t) + 1\right]\right\} \qquad (4)$$

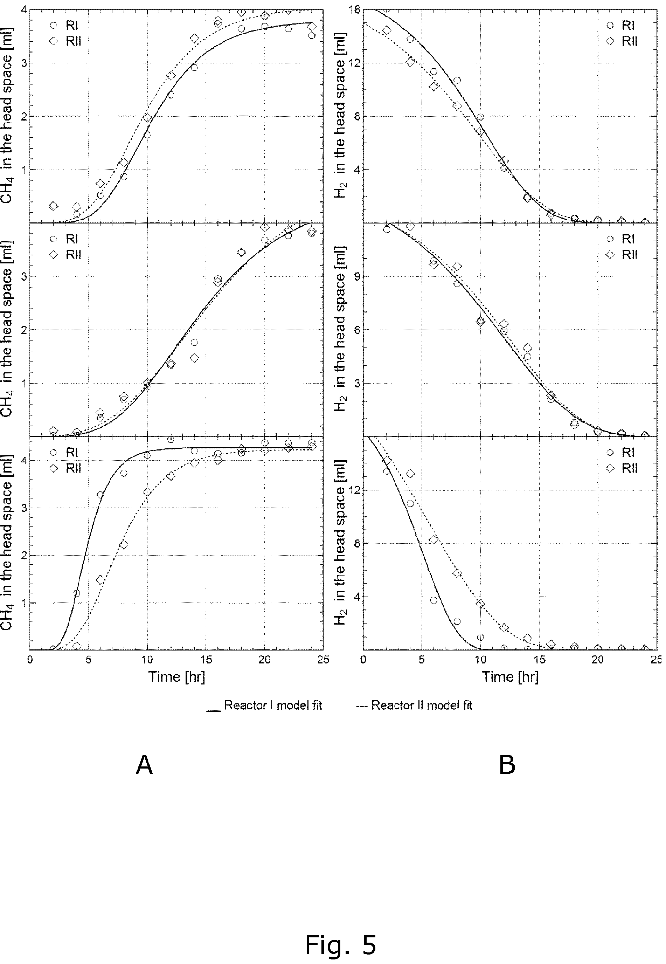

Where y represents the component formed or consumed (Methane or Hydrogen) (ml), $A$ is the maximum product formed (ml), $\mu_m$ is the maximum rate of production (ml/hr), $\lambda$ is the lag time to exponential product formation (hr), and $t$ is the fermentation time (hr). The fitted curves for methane and hydrogen are presented in Figures 5(a) and 5(b). The correlation coefficients were above 0.96 in all cases suggesting the suitability of the modified Gompertz model to describe the formation of methane and consumption of hydrogen by Hydrogenotrophic methanogen archaea groups during batch fermentation.

*Microbial community in ex-situ biogas upgrading membrane bioreactors*

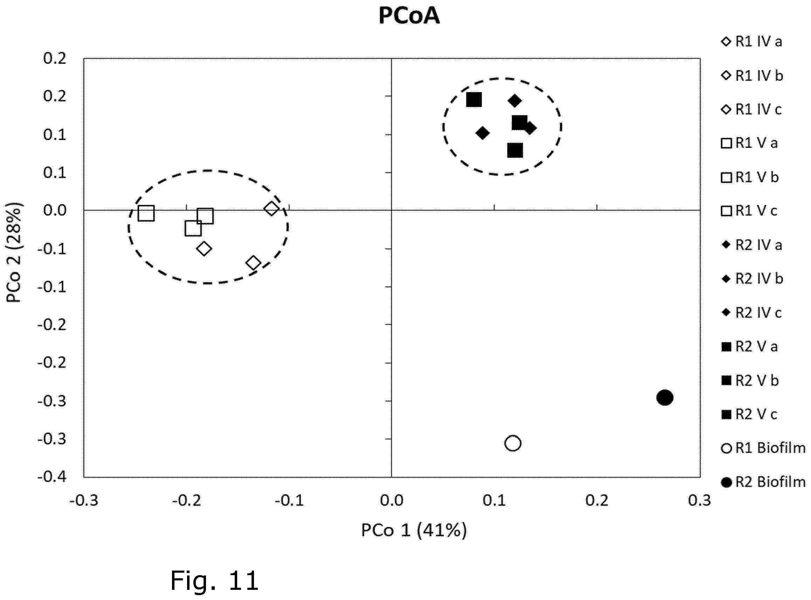

The sequencing generated more than 3 million raw reads with an average length of 252 bp corresponding to nearly 96 thousand paired sequences per sample after a quality improving process. Alpha diversity based on the number of OTUs confirmed that the sequencing depth was enough to cover the microbial species richness. The principal component analysis (PcoA) indicated that the microbial community patterns varied among the reactors (Figure 11) and a considerable variation was also noted between the digesters liquid and biofilm samples. Finally, the taxonomic assignment of the most abundant OTUs is given in Table 2.

Table 2. Microbial community relative abundance data

| Microbial name | Relative Abundance (%) | | | | | |
|---|---|---|---|---|---|---|
| | R1 phase IV | R1 phase V | R2 phase IV | R2 phase V | R1 Biofilm | R2 Biofilm |
| **Methanothermobacter thermautotrophicus 1** | 7.74 | 5.32 | 13.12 | 11.19 | 2.15 | 3.08 |
| **Methanobacterium formicicum 2** | 0.00 | 0.00 | 0.11 | 2.70 | 0.00 | 18.88 |
| **Methanoculleus palmolei 3** | 0.22 | 0.12 | 0.07 | 0.04 | 4.34 | 1.05 |
| **Anaerobaculum mobile 4** | 11.30 | 8.66 | 14.47 | 17.54 | 8.93 | 18.27 |
| **Clostridia sp. 5** | 12.97 | 14.98 | 13.00 | 12.12 | 5.00 | 5.42 |
| **Clostridia sp. 6** | 9.86 | 9.37 | 12.06 | 9.19 | 6.84 | 6.87 |
| **Bacteroidales sp. 7** | 7.18 | 5.82 | 11.34 | 8.14 | 13.33 | 5.82 |
| **Thermacetogenium sp. 8** | 17.04 | 21.23 | 0.99 | 1.45 | 2.32 | 2.35 |
| **Natronincola peptidivorans 9** | 2.61 | 2.57 | 3.98 | 1.01 | 28.66 | 2.66 |
| **Bacillales sp. 10** | 2.41 | 2.07 | 2.70 | 3.48 | 1.46 | 1.12 |
| **Campylobacteraceae sp. 11** | 2.94 | 2.80 | 0.63 | 1.76 | 3.76 | 0.29 |
| **Pseudomonas sp. 12** | 1.30 | 0.96 | 3.48 | 3.93 | 0.53 | 0.56 |
| **Clostridia sp. 13** | 2.44 | 4.00 | 0.95 | 0.87 | 1.11 | 0.32 |
| **Gammaproteobacteria sp. 14** | 1.21 | 1.46 | 1.46 | 1.58 | 0.52 | 0.04 |
| **Anaerobrancaceae sp. 15** | 1.62 | 1.62 | 0.75 | 1.85 | 0.29 | 0.15 |
| **Clostridia sp. 16** | 1.42 | 2.07 | 0.86 | 0.57 | 0.35 | 0.17 |
| **Lascolabacillus massiliensis 17** | 0.31 | 0.40 | 0.24 | 0.81 | 0.85 | 2.11 |
| **Thermoanaerobactera ceae sp. 18** | 0.81 | 0.60 | 0.82 | 0.79 | 0.81 | 0.86 |
| **Thermacetogenium sp. 19** | 0.88 | 1.22 | 0.22 | 1.71 | 0.20 | 0.36 |
| **Thermoanaerobactera ceae sp. 20** | 0.48 | 0.22 | 1.08 | 0.60 | 0.70 | 1.10 |
| **Tepidimicrobium sp. 21** | 0.23 | 0.21 | 0.63 | 1.68 | 0.07 | 1.27 |
| **Thermacetogenium sp. 22** | 0.61 | 0.41 | 0.59 | 0.79 | 0.63 | 0.40 |
| **Anaerovirgula sp. 23** | 0.05 | 0.08 | 1.07 | 0.33 | 0.67 | 1.07 |
| **Pseudomonas sp. 24** | 1.77 | 1.01 | 0.09 | 0.07 | 0.16 | 0.08 |
| **Bacteroidales sp. 25** | 0.50 | 0.11 | 0.22 | 0.62 | 0.23 | 1.09 |

(continued)

| Microbial name | Relative Abundance (%) | | | | | |
|---|---|---|---|---|---|---|
| | R1 phase IV | R1 phase V | R2 phase IV | R2 phase V | R1 Biofilm | R2 Biofilm |
| Tepidimicrobium sp. 26 | 0.34 | 0.60 | 0.03 | 0.03 | 1.58 | 0.17 |
| Firmicutes sp. 27 | 0.28 | 0.42 | 0.72 | 0.64 | 0.22 | 0.07 |
| Clostridia sp. 28 | 0.07 | 0.10 | 0.08 | 0.11 | 0.69 | 1.28 |
| Thermoanaerobactera ceae sp. 29 | 0.28 | 0.39 | 0.46 | 0.64 | 0.14 | 0.15 |
| Anaerovirgula multivorans 30 | 0.15 | 0.11 | 0.08 | 0.19 | 1.26 | 0.26 |
| Bacillus cytotoxicus 31 | 0.00 | 0.00 | 0.20 | 0.15 | 0.00 | 1.64 |
| Bacillales sp. 32 | 0.13 | 0.35 | 1.07 | 0.22 | 0.02 | 0.01 |
| Caldicoprobacteraceae sp. 33 | 0.11 | 0.10 | 0.34 | 0.25 | 0.35 | 0.63 |
| Sinibacillus 34 | 0.37 | 0.36 | 0.62 | 0.28 | 0.04 | 0.01 |
| Alkaliphilus 35 | 0.00 | 0.00 | 0.00 | 0.01 | 0.00 | 1.60 |
| Bacteroidales sp. 36 | 0.08 | 0.03 | 0.08 | 0.31 | 0.34 | 0.73 |
| [Bacillus] thermocloacae 37 | 0.21 | 0.27 | 0.48 | 0.22 | 0.19 | 0.05 |
| Bacillus andreesenii 38 | 0.00 | 0.00 | 0.65 | 0.12 | 0.00 | 0.58 |
| Clostridia sp. 39 | 0.30 | 0.47 | 0.14 | 0.11 | 0.08 | 0.08 |
| Haloplasmataceae sp. 40 | 0.08 | 0.05 | 0.33 | 0.55 | 0.01 | 0.01 |
| Tepidimicrobium 41 | 0.05 | 0.14 | 0.01 | 0.01 | 0.81 | 0.00 |
| Porphyromonadaceae sp. 42 | 0.03 | 0.06 | 0.05 | 0.56 | 0.04 | 0.17 |
| Proteiniborus ethanoligenes 43 | 0.05 | 0.05 | 0.01 | 0.01 | 0.76 | 0.02 |
| Thermoanaerobacterales sp. 44 | 0.03 | 0.03 | 0.03 | 0.06 | 0.09 | 0.66 |
| Bacillus sp. 45 | 0.00 | 0.00 | 0.25 | 0.40 | 0.00 | 0.06 |

The portion of the bacterial population in the anaerobic reactors was on average 87% of the total microbial community. The most dominant phyla were *Firmicutes* (46.1%), Synergistetes (13.2%), *Euryarchaeota* (11.7%), *Bacteroidetes* (10.3%) and *Proteobacteria* (5.4%) while *OP9 division* and *Tenericutes* were identified at lower proportions. The most abundant microbe (average abundance in all samples equal to 13.2%) belonged to the bacterial species *Anaerobaculum mobile.* This strictly anaerobic and motile bacterium was first isolated from a wool-scouring wastewater treatment lagoon. It ferments glucose, organic acids, glycerol and peptides to acetate, $H_2$ and $CO_2$. However, the reason for high prevalence of *A. mobile* in reactors fed mainly gaseous substrates ($H_2$ and $CO_2$) is not apparent. The second and third most abundant OTU's (Clostridia sp. 5, Clostridia sp. 6) were an uncultured species affiliated to the orders MBA08 and SHA-98 of the class Clostridia respectively.

The microbial community in a membrane bioreactor predominantly exists in a suspended form or attached to the membrane surface as biofilm. However, they could also switch between suspended and attached modes in response to the prevailing fluid hydrodynamics. The diversity within the reactor liquids and biofilms suggested that the community in the liquid samples were highly diverse than the biofilm samples. Hence, OTU's belonging *Clostridia sp. 5, Clostridia sp. 6, Thermacetogenium sp. 8* and *M. thermautotrophicus 1* were predominantly found suspended in the reactor liquid. However, *A. mobile* (12.99-13.6%) and *Bacteroidales sp. 7* (8.12-9.58%) species were prevalent in both environments.

Nevertheless, the biofilm was dominated by OTU's affiliated to *Methanobacterium formicicum 2* and *Natronincola peptidivorans 9.*

The archaeal domain represented 13.0% of the overall microbial diversity; moreover, the main species belonged to *Methanothermobacter thermautotrophicus, Methanobacterium formicicum* and *Methanoculleus palmolei.* The predominant hydrogenotrophic archaea *M. thermautotrophicus* accounted on average 91% of the archaeal species in the reactors broth; however, its proportion within the biofilms accounted only around 17%. In contrast, *M. formicicum* selectively dominated the biofilm in reactor II; while, *M. palmolei* was more prevalent within the biofilm system of reactor I. In general, the relative abundance data indicated that the biofilm system inhabited around 60% of the archeal communities identified in both membrane bioreactors. This indicates the importance of biofilm system in supporting the archeal communities, which are primarily responsible for hydrogenotrophic methanogenesis based biogas upgrading process.

*Conclusions*

A membrane bioreactor configuration that constitute a continuous stirred tank reactor coupled with membrane gas liquid contactor module was successfully operated to effect microbial assisted conversion of a stoichiometric mixture of carbon dioxide and hydrogen into methane gas. Results show that Reactor I that represent a single strand tubular membrane bioreactor achieved an upgraded biogas of 98% $CH_4$ content. While that of Reactor II, which employed a capillary membrane achieved up to 99% $CH_4$ content. The study demonstrated that Ex-situ biogas upgrading is possible using anaerobic membrane bioreactor system and in this system operation without gas recirculation gave positive results as can be observed in the reactors performance during stages III-V. There was comparable methanogenic activity between the two reactors regarding Hydrogen gas consumption and subsequent methane production; however, there was marked difference in the activity of the reactors in stage IV. This could be attributed to the larger surface area of reactor I that facilitated better gas liquid mass transfer and subsequent microbial adaptation and rise in microbial activity.
In addition, the microbial community study indicated that there exists considerable diversity variation between the reactor liquors and biofilm systems. Similarly, the archeal communities were predominantly found within the biofilm system, which indicates the vital role biofilm system plays in Ex-situ biogas upgrading process.

**Claims**

1.   A system (100) for increasing the methane content of biogas, the system comprising

   - a biogas reactor (1) capable of generating biogas; and
   - a membrane biofilm reactor (9) arranged for converting carbon dioxide ($CO_2$) in the biogas and supplied hydrogen ($H_2$) into methane ($CH_4$), the membrane biofilm reactor being arranged for comprising a biofilm of methanogens (21) on or in, the membrane (20), said membrane comprising a first side and a second side;

   wherein the membrane biofilm reactor (9) is positioned separately and outside of the biogas reactor (1), the biogas reactor being in fluid connection with the membrane biofilm reactor (9) by:

      ▪ a fluid intake line (6) for circulating fluid from the biogas reactor (1) to said first side of the membrane (20) so as to provide nutrients to the biofilm and for absorbing the methane gas generated by the biofilm of methanogens (21); and
      ▪ a fluid return line (11) in fluid connection with said first side of the membrane for returning the methane enriched fluid from the membrane biofilm reactor (9) to the biogas reactor (1).

2.   The system according to claim 1, wherein a supply line (17, 18) is arranged for conveying gas, from the biogas reactor (1) to the membrane biofilm reactor (9), said supply line being different from said liquid intake line (6) and said supply line (17,18) is particularly arranged for conveying gas, to said second side of the membrane (20) in said membrane biofilm reactor (9) and said supply line (17, 18) is in fluid connection with a hydrogen gas ($H_2$) source (15), preferably an external hydrogen gas source, capable of regulating the amount of hydrogen in the supply line (17, 18).

3.   The system according to any of the preceding claims, wherein the membrane is hydrophobic, preferably a polysulphone membrane.

4.   The system according to any of the preceding claims, wherein the membrane (20) on the first side comprises a biofilm attachment promoting surface.

5.   The system according to any of the preceding claims, wherein the methanogens are hydrolytic, acidogenic, acetogenic and/or methanogenic microorganisms.

6.   The system according to any of the preceding claims, wherein the membrane biofilm reactor comprises a biofilm (21) of methanogens on or in, the membrane.

7. The system according to any of the preceding claims, wherein the biogas reactor comprises a liquid or semiliquid digestate and a gas phase comprising biogas.

8. The system according to any of the preceding claims, wherein the system does not comprise a membrane biofilm reactor inside a biogas reactor.

9. The system according to any of the preceding claims, wherein the biofilm is supplied with exogenously supplied $H_2$ outside a biogas reactor.

10. The system according to any of the preceding claims, wherein the system is adapted to allow for repair and/or shift of all or part of the membrane biofilm reactor, without having to access a biogas reactor.

11. A method for increasing the methane content of biogas, the method comprising

- generating biogas in a biogas reactor (1); and
- converting carbon dioxide ($CO_2$) in the biogas and supplied hydrogen ($H_2$) into methane ($CH_4$) in a membrane biofilm reactor, the membrane biofilm reactor comprising a biofilm of methanogens (21) on or in, a membrane (20), said membrane comprising a first side and a second side;

the membrane biofilm reactor (9) being positioned separately and outside of the biogas reactor (1), the biogas reactor being in fluid connection with the membrane biofilm reactor (9) by:

▪ a fluid intake line (6) for circulating fluid from the biogas reactor (1) to said first side of the membrane (20) so as to provide nutrients to the biofilm and for absorbing the methane gas generated by the biofilm of methanogens (21); and
▪ a fluid return line (11) in fluid connection with said first side of the membrane for returning the methane enriched fluid from the membrane biofilm reactor (9) to the biogas reactor (1).

12. The method according to claim 11, wherein the pressure on gas side of the membrane is higher than the pressure on the liquid side of the membrane.

13. The method according to claim 11 or 12, wherein

- collected upgraded biogas has a methane content above 95% by volume, such as above 97%, such as above 98% such as above 99%, or such as above 99.5%; and/or
- collected upgraded biogas has a methane content above 98% by volume, such as above 99%, or such as above 99.5%; and/or
- collected biogas has a $H_2$ content below 2.2% by volume, such as below 2%, preferably below 1.5%, more preferably below 1%, such as below 0.8%; and/or
- the amount of $CO_2$ in collected biogas is below 2.0% by volume, such as below 1.5%, preferably below 1.0%, more preferably below 0.5%, such as below 0.3%.

14. The method according to any of claims 11-13, wherein the bioreactor operates at a pH above 8.4, such as above 8.6, such as above 8.8, or such as in the range 8.4-8.9, such as in the range 8.5-8.9 or in the range 8.6 - 8.9.

15. A computer program product being adapted to enable a computer system comprising at least one computer having data storage means in connection therewith arranged for performing the method according to any of claims 11-14, implemented in a system according to any of claim 1-10.

**Patentansprüche**

1. System (100) zum Erhöhen des Methangehalts von Biogas, wobei das System Folgendes umfasst:

- einen Biogasreaktor (1), der dazu in der Lage ist, Biogas zu erzeugen; und
- einen Membran-Biofilmreaktor (9), der zum Umwandeln von Kohlendioxid ($CO_2$) in dem Biogas und bereitgestelltem Wasserstoff ($H_2$) zu Methan ($CH_4$) angeordnet ist, wobei der Membran-Biofilmreaktor dazu angeordnet ist, einen Biofilm aus Methanogenen (21) an oder in der Membran (20) zu umfassen, wobei die Membran eine erste Seite und eine zweite Seite umfasst;

wobei der Membran-Biofilmreaktor (9) separat und außerhalb des Biogasreaktors (1) angeordnet ist, wobei der Biogasreaktor durch Folgendes mit dem Membran-Biofilmreaktor (9) in Fluidverbindung steht:

- eine Fluideinlassleitung (6) zum Zirkulieren von Fluid aus dem Biogasreaktor (1) zu der ersten Seite der Membran (20), um so dem Biofilm Nährstoffe bereitzustellen, und zum Absorbieren des durch den Biofilm aus Methanogenen (21) erzeugten Methangases; und
- eine Fluidrückführleitung (11) in Fluidverbindung mit der ersten Seite der Membran zum Rückführen des mit Methan angereicherten Fluids von dem Membran-Biofilmreaktor (9) zu dem Biogasreaktor (1).

2. System nach Anspruch 1, wobei eine Versorgungsleitung (17, 18) zum Befördern von Gas von dem Biogasreaktor (1) zu dem Membran-Biofilmreaktor (9) angeordnet ist, wobei sich die Versorgungsleitung von der Flüssigkeitseinlassleitung (6) unterscheidet und die Versorgungsleitung (17,18) insbesondere zum Befördern von Gas zu der zweiten Seite der Membran (20) in dem Membran-Biofilmreaktor (9) angeordnet ist und die Versorgungsleitung (17, 18) mit einer Quelle (15) von Wasserstoffgas ($H_2$), bevorzugt einer externen Wasserstoffgasquelle, die in der Lage ist, die Menge von Wasserstoff in der Versorgungsleitung (17, 18) zu regulieren, in Fluidverbindung steht.

3. System nach einem der vorhergehenden Ansprüche, wobei die Membran hydrophob, bevorzugt eine Polysulphonmembran, ist.

4. System nach einem der vorhergehenden Ansprüche, wobei die Membran (20) an der ersten Seite eine die Biofilmanhaftung fördernde Oberfläche umfasst.

5. System nach einem der vorhergehenden Ansprüche, wobei die Methanogene hydrolytische, säurebildende, acetogene und/oder methanogene Mikroorganismen sind.

6. System nach einem der vorhergehenden Ansprüche, wobei der Membran-Biofilmreaktor einen Biofilm (21) aus Methanogenen an oder in der Membran umfasst.

7. System nach einem der vorhergehenden Ansprüche, wobei der Biogasreaktor einen flüssigen oder halbflüssigen Gärrückstand und eine Biogas-umfassende Gasphase umfasst.

8. System nach einem der vorhergehenden Ansprüche, wobei das System keinen Membran-Biofilmreaktor innerhalb eines Biogasreaktors umfasst.

9. System nach einem der vorhergehenden Ansprüche, wobei der Biofilm mit exogen bereitgestelltem $H_2$ außerhalb eines Biogasreaktors versorgt wird.

10. System nach einem der vorhergehenden Ansprüche, wobei das System dazu angepasst ist, Reparatur und/oder Wechsel der Gesamtheit oder eines Teils des Membran-Biofilmreaktors zu ermöglichen, ohne dabei auf einen Biogasreaktor zugreifen zu müssen.

11. Verfahren zum Erhöhen des Methangehalts von Biogas, wobei das Verfahren Folgendes umfasst:

- Erzeugen von Biogas in einem Biogasreaktor (1); und
- Umwandeln von Kohlendioxid ($CO_2$) in dem Biogas und bereitgestelltem Wasserstoff ($H_2$) zu Methan ($CH_4$) in einem Membran-Biofilmreaktor, wobei der Membran-Biofilmreaktor einen Biofilm aus Methanogenen (21) an oder in einer Membran (20) umfasst, wobei die Membran eine erste Seite und eine zweite Seite umfasst;

wobei der Membran-Biofilmreaktor (9) separat und außerhalb des Biogasreaktors (1) angeordnet ist, wobei der Biogasreaktor durch Folgendes mit dem Membran-Biofilmreaktor (9) in Fluidverbindung steht:

- eine Fluideinlassleitung (6) zum Zirkulieren von Fluid aus dem Biogasreaktor (1) zu der ersten Seite der Membran (20), um so dem Biofilm Nährstoffe bereitzustellen, und zum Absorbieren des durch den Biofilm aus Methanogenen (21) erzeugten Methangases; und
- eine Fluidrückführleitung (11) in Fluidverbindung mit der ersten Seite der Membran zum Rückführen des mit Methan angereicherten Fluids von dem Membran-Biofilmreaktor (9) zu dem Biogasreaktor (1).

**12.** Verfahren nach Anspruch 11, wobei der Druck an der Gasseite der Membran höher als der Druck an der Flüssigkeitsseite der Membran ist.

**13.** Verfahren nach Anspruch 11 oder 12, wobei

- gesammeltes aufgewertetes Biogas einen Methangehalt über 95 Volumen-% aufweist, wie etwa über 97 %, wie etwa über 98 %, wie etwa über 99 % oder wie etwa über 99,5 %; und/oder
- gesammeltes aufgewertetes Biogas einen Methangehalt über 98 Volumen-% aufweist, wie etwa über 99 % oder wie etwa über 99,5 %; und/oder
- gesammeltes Biogas einen $H_2$-Gehalt unter 2,2 Volumen-% aufweist, wie etwa unter 2 %, bevorzugt unter 1,5 %, bevorzugter unter 1 %, wie etwa unter 0,8 %; und/oder
- die Menge von $CO_2$ in gesammeltem Biogas unter 2,0 Volumen-% liegt, wie etwa unter 1,5 %, bevorzugt unter 1,0 %, bevorzugter unter 0,5 %, wie etwa unter 0,3 %.

**14.** Verfahren nach einem der Ansprüche 11-13, wobei der Bioreaktor bei einem pH-Wert über 8,4 betrieben wird, wie etwa über 8,6, wie etwa über 8,8 oder wie etwa in dem Bereich 8,4-8,9, wie etwa in dem Bereich 8,5-8,9 oder in dem Bereich 8,6-8,9.

**15.** Computerprogrammprodukt, das dazu angepasst ist, es einem Computersystem, das zumindest einen Computer umfasst, bei dem Datenspeichermittel in Verbindung damit zum Durchführen des Verfahrens nach einem der Ansprüche 11-14 angeordnet sind, zu ermöglichen, in einem System nach einem der Ansprüche 1-10 umgesetzt zu werden.

**Revendications**

**1.** Système (100) permettant d'augmenter la teneur en méthane d'un biogaz, le système comprenant

- un réacteur de biogaz (1) pouvant générer un biogaz ; et
- un réacteur de membrane à biofilm (9) conçu pour convertir le dioxyde de carbone ($CO_2$) dans le biogaz et l'hydrogène ($H_2$) alimenté en méthane ($CH_4$), le réacteur de membrane à biofilm étant conçu pour comprendre un biofilm de méthanogènes (21) sur ou dans la membrane (20), ladite membrane comprenant un premier côté et un second côté ;

ledit réacteur à biofilm à membrane (9) étant disposé séparément et à l'extérieur du réacteur de biogaz (1), le réacteur de biogaz étant en connexion fluidique avec le réacteur de membrane à biofilm (9) par l'intermédiaire de :

- une ligne d'admission de fluide (6) pour faire circuler un fluide du réacteur de biogaz (1) audit premier côté de la membrane (20) de façon à fournir des nutriments au biofilm et pour absorber le gaz de méthane généré par le biofilm de méthanogènes (21) ; et
- une ligne de retour de fluide (11) en connexion fluidique avec ledit premier côté de la membrane pour renvoyer le fluide enrichi en méthane du réacteur à biofilm à membrane (9) au réacteur de biogaz (1).

**2.** Système selon la revendication 1, dans lequel une ligne d'alimentation (17, 18) est disposée pour acheminer un gaz du réacteur de biogaz (1) au réacteur de membrane à biofilm (9), ladite ligne d'alimentation étant différente de ladite ligne d'admission de liquide (6) et ladite ligne d'alimentation (17, 18) étant conçue tout particulièrement pour acheminer un gaz vers ledit second côté de la membrane (20) dans ledit réacteur de membrane à biofilm (9) et ladite ligne d'alimentation (17, 18) étant en connexion fluidique avec une source de gaz d'hydrogène ($H_2$) (15), de préférence une source de gaz d'hydrogène externe, pouvant réguler la quantité d'hydrogène dans la ligne d'alimentation (17, 18).

**3.** Système selon l'une quelconque des revendications précédentes, dans lequel ladite membrane est hydrophobe, de préférence une membrane de polysulfone.

**4.** Système selon l'une quelconque des revendications précédentes, dans lequel ladite membrane (20) sur le premier côté comprend une surface favorisant l'attachement d'un biofilm.

**5.** Système selon l'une quelconque des revendications précédentes, dans lequel lesdits méthanogènes sont des microorganismes hydrolytiques, acidogènes, acétogènes et/ou méthanogènes.

6. Système selon l'une quelconque des revendications précédentes, dans lequel ledit réacteur de membrane à biofilm comprend un biofilm (21) de méthanogènes sur ou dans la membrane.

7. Système selon l'une quelconque des revendications précédentes, dans lequel ledit réacteur de biogaz comprends un digestat liquide ou semi-liquide et une phase gazeuse comprenant un biogaz.

8. Système selon l'une quelconque des revendications précédentes, ledit système ne comprenant pas de réacteur de membrane à biofilm à l'intérieur d'un réacteur de biogaz.

9. Système selon l'une quelconque des revendications précédentes, ledit biofilm étant alimenté avec du $H_2$ alimenté de manière exogène à l'extérieur d'un réacteur de biogaz.

10. Système selon l'une quelconque des revendications précédentes, ledit système étant conçu pour permettre la réparation et/ou le changement de la totalité ou d'une partie du réacteur de membrane à biofilm, sans avoir à accéder à un réacteur de biogaz.

11. Procédé permettant d'augmenter la teneur en méthane d'un biogaz, le procédé comprenant

   - la génération d'un biogaz dans un réacteur de biogaz (1) ; et
   - la conversion du dioxyde de carbone ($CO_2$) en le biogaz et de l'hydrogène ($H_2$) alimenté en méthane ($CH_4$) dans un réacteur de membrane à biofilm, le réacteur de membrane à biofilm comprenant un biofilm de méthanogènes (21) sur ou dans une membrane (20), ladite membrane comprenant un premier côté et un second côté ;

   ledit réacteur de membrane à biofilm (9) étant disposé séparément et à l'extérieur du réacteur de biogaz (1), le réacteur de biogaz étant en connexion fluidique avec le réacteur de membrane à biofilm (9) par l'intermédiaire de :

   ■ une ligne d'admission de fluide (6) pour faire circuler un fluide du réacteur de biogaz (1) audit premier côté de la membrane (20) de façon à fournir des nutriments au biofilm et pour absorber le gaz de méthane généré par le biofilm de méthanogènes (21) ; et
   ■ une ligne de retour de fluide (11) en connexion fluidique avec ledit premier côté de la membrane pour renvoyer le fluide enrichi en méthane du réacteur à biofilm à membrane (9) au réacteur de biogaz (1).

12. Procédé selon la revendication 11, la pression du côté gaz de la membrane étant supérieure à la pression du côté liquide de la membrane.

13. Procédé selon la revendication 11 ou 12, dans lequel

   - le biogaz enrichi recueilli présente une teneur en méthane supérieure à 95 % en volume, telle que supérieure à 97 %, telle que supérieure à 98 %, telle que supérieure à 99 %, ou telle que supérieure à 99,5 % ; et/ou
   - le biogaz enrichi recueilli présente une teneur en méthane supérieure à 98 % en volume, telle que supérieure à 99 %, ou telle que supérieure à 99,5 % ; et/ou
   - le biogaz recueilli présente une teneur en $H_2$ inférieure à 2,2 % en volume, telle qu'inférieure à 2 %, de préférence inférieure à 1,5 %, plus préférablement inférieure à 1 %, telle qu'inférieure à 0,8 % ; et/ou
   - la quantité de $CO_2$ dans le biogaz recueilli est inférieure à 2,0 % en volume, telle qu'inférieure à 1,5 %, de préférence inférieure à 1,0 %, plus préférablement inférieure à 0,5 %, telle qu'inférieure à 0,3 %.

14. Procédé selon l'une quelconque des revendications 11 à 13, ledit bioréacteur fonctionnant à un pH supérieur à 8,4, tel que supérieur à 8,6, tel que supérieur à 8,8, ou tel que compris dans la plage de 8,4 à 8,9, tel que compris dans la plage de 8,5 à 8,9 ou dans la plage de 8,6 à 8,9.

15. Produit de programme informatique conçu pour permettre à un système informatique comprenant au moins un ordinateur doté d'un moyen de stockage de données en connexion avec celui-ci d'exécuter le procédé selon l'une quelconque des revendications 11 à 14, mis en œuvre dans un système selon l'une quelconque des revendications 1 à 10.

Fig. 1

EP 3 555 258 B1

Fig. 2

Fig. 3

EP 3 555 258 B1

Fig. 4

24

A          B

Fig. 5

Fig. 6, continues

(c)

(d)

(e)

Biofilm 21
Porous membrane 20

Biofilm 21
Non porous membrane 20

Biofilm 21
Composite membrane 20

Fig. 6, continued

Membrane 20
Biofilm 21
Liquid phase

CH$_4$/Upgraded biogas

H$_2$, CO$_2$/Biogas
Feed gas

Fig. 7

**CH$_4$/Upgraded biogas**

H$_2$, CO$_2$/Biogas
Feed gas

Membrane 20

Biofilm 21

Liquid phase

**CH$_4$/Upgraded biogas**

Fig. 8

Fig. 9

Fig. 10

Fig. 11

EP 3 555 258 B1

**EP 3 555 258 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014342426 A
- US 2009286296 A
- US 5779897 A